(19) Europäisches Patentamt
European Patent Office
Office européen des brevets

(11) Veröffentlichungsnummer: **0 401 505 A2**

(12) # EUROPÄISCHE PATENTANMELDUNG

(21) Anmeldenummer: **90108032.5**

(51) Int. Cl.[5]: **A61K 45/06, A61K 37/64**

(22) Anmeldetag: **27.04.90**

Die Bezeichnung der Erfindung wurde geändert (Richtlinien für die Prüfung im EPA, A-III, 7.3).

(30) Priorität: **10.05.89 DE 3915236**

(43) Veröffentlichungstag der Anmeldung: **12.12.90 Patentblatt 90/50**

(84) Benannte Vertragsstaaten: **AT CH DE FR GB IT LI**

(71) Anmelder: **MERCK PATENT GESELLSCHAFT MIT BESCHRÄNKTER HAFTUNG**
**Frankfurter Strasse 250**
**D-6100 Darmstadt(DE)**

(72) Erfinder: **Gericke, Rolf, Dr.**
**Mozartstrasse 19**
**D-6104 Seeheim(DE)**
Erfinder: **Baumgarth, Manfred, Dr.**
**Sachsenstrasse 53**
**D-6100 Darmstadt(DE)**
Erfinder: **Lues, Inge, Dr.**
**Paul Wagnerstrasse 13**
**D-6100 Darmstadt(DE)**
Erfinder: **Bergmann, Rolf, Dr.**
**Birkenhag 36**
**D-6101 Reichelsheim(DE)**
Erfinder: **De Peyer, Jacques, Dr.**
**Weisenbühlweg 33K**
**CH-3007 Bern(DE)**

(54) **Pharmazeutische Zubereitung mit einem Kaliumkanalaktivator und einem ACE-Hemmer.**

(57) Die Erfindung betrifft eine pharmazeutische Zubereitung enthaltend einen ACE-Hemmer und einen Kaliumkanalaktivator der Formel I

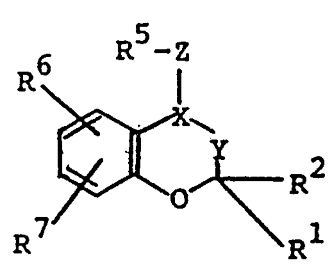

I

worin
X-Y, $R^1$, $R^2$, $R^5$, $R^6$, $R^7$ und Z die in Patentanspruch 1 angegebene Bedeutung haben.

EP 0 401 505 A2

## Pharmazeutische Zubereitung

Gegenstand der Erfindung ist eine neue pharmazeutische Zubereitung enthaltend einen Kaliumkanalaktivator und einen ACE-Hemmer, dadurch gekennzeichnet, daß sie einen Kaliumkanalaktivator der Formel I enthält:

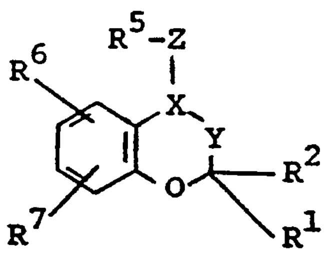

I

worin

X-Y =CH-CHR$^8$-, =CR$^4$-CR$^3$A- oder =CH-CA(OA)-, sowie, falls der Rest R$^5$ weder vollständig noch partiell hydriert ist, auch =CH$^4$-CHR$^3$- oder =CH-CH(OA)-,

R' A,

R$^2$ und R$^8$ jeweils H oder A,

R' und R$^2$ zusammen auch Alkylen mit 3-6 C-Atomen,

R$^3$ OH oder OAc,

R$^4$ H,

R$^3$ und R$^4$ zusammen auch eine Bindung,

R$^5$ einen unsubstituierten oder einen ein- oder zweifach durch A, F, Cl, Br, J, OH, OA, OAc, $NO_2$, $NH_2$, AcNH, HOOC und/oder AOOC substituierten Pyridyl-, Pyridazinyl-, Pyrimidinyl-, Pyrazinyl-, Oxo-dihydropyridyl-, Oxo-dihydropyridazinyl-, Oxo-dihydro-pyrimidinyl-, Oxo-dihydro-pyrazinyl-, 3H- oder 5H-2-Pyrrolinon-1-yl-, 2H-1-Isochinolinon-2-yl-, 2H-1-Phthalazinon-2-yl-, 3H-4-Chinazolinon-3-yl- oder 1H-2-Thiopyridon-1-yl-rest, wobei diese Reste auch vollständig oder partiell hydriert sein können,

R$^6$ und R$^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C(=$NNH_2$),

Z eine Bindung, O, S oder NH,

R$^7$ auch Pyridyl, Pyridazinyl, Pyrimidinyl oder Pyrazinyl,

A Alkyl mit 1-6 C-Atomen (wobei mehrere Gruppen A unabhängig voneinander verschiedene Alkylgruppen bedeuten können),

-alkyl- Alkylen mit 1-6 C-Atomen und

Ac Alkanoyl mit 1-8 C-Atomen oder Aroyl mit 7-11 C-Atomen

bedeuten,

und/oder eines seiner physiologisch unbedenklichen Salze.

Ähnliche pharmazeutische Zubereitungen sind in der EP-A2-02 71 271 beschrieben.

Die Verbindungen der Formel I sind teilweise bekannt (vgl. z.B. DE-A-37 26 261).

Unter den Verbindungen der Formel I sind diejenigen bevorzugt, in denen die Reste R' und R$^2$ jeweils $CH_3$, der Rest R$^7$ H und/oder der Rest R$^6$ CN bedeuten, wobei der Rest R$^6$ vorzugsweise in 6-Stellung steht. Die Gruppierung X-Y bedeutet vorzugsweise =CR$^4$-CR$^3$A- [insbesondere =CH-C(OH)A- oder =C=CA-] oder, falls der Rest R$^5$ weder vollständig noch partiell hydriert ist, auch =CR$^4$-CHR$^3$- (insbesondere =CH-CHOH- oder =C=CH-).

Die Gruppe R$^5$-Z bedeutet vorzugsweise 1H-2-Pyridon-1-yl, 2-Hydroxy-4-pyridyl-oxy, 6-Hydroxy-3-pyridazinyl-oxy, 1,6-Dihydro-1-methyl- oder 1,6-Dihydro-1-ethyl-6-oxo-3-pyridazinyl-oxy.

Einzelne bevorzugte Verbindungen der Formel I sind 2,2-Dimethyl-4-(1H-2-pyridon-1-yl)-6-cyan-3-chromanol (Ia), insbesondere dessen (-)-Enantiomeres, 2,2-Dimethyl-4-(1H-2-pyridon-1-yl)-6-cyan-2H-chromen (Ib), 2,2-Dimethyl-4-(6-hydroxy-3-pyridazinyl-oxy)-6-cyan-3-chromanol, insbesondere dessen (-)-Enantiomeres, 2,2,3-Trimethyl-4-(6-hydroxy-3-pyridazinyl-oxy)-6-cyan-3-chromanol, insbesondere dessen (-)-Enantiomeres, 2,2-Dimethyl-4-(1,6-dihydro-1-methyl-6-oxo-3-pyridazinyl-oxy)-6-cyan-3-chromanol (Ic), insbesondere

dessen (-)-Enantiomeres.

ACE (= angiotensin converting enzyme)-Hemmer sind Substanzen, die die Wirkung von Enzymen hemmen, welche Angiotensin I in Angiotensin II umwandeln. ACE-Hemmer senken den Blutdruck, wenn der Bluthochdruck auf Angiotensin II zurückgeführt werden kann. Als Vasodilatatoren weisen sie ein günstiges hämodynamisches Profil auf.

Bevorzugte ACE-Hemmer sowie Verfahren zu ihrer Herstellung sind z. B. in der DE-OS 33 17 290 und der EP-OS 12 401 angegeben. Es seien z. B. diejenigen folgender Formel genannt:

$R\text{-}CO\text{-}CR^1R^2\text{-}NH\text{-}CHR^3\text{-}CO\text{-}NR^4\text{-}CR^5R^7\text{-}CO\text{-}R^6$

worin

R und $R^6$ gleich oder verschieden sind und Hydroxy, Niedrigalkoxy, Niedrigalkenoxy, Di-niedrigalkylamino-niedrigalkoxy, Acylamino-niedrigalkoxy, Acyloxy-niedrigalkoxy, Aryloxy, Ar-niedrigalkoxy, substituiertes Aryloxy oder substituiertes Ar-niedrigalkoxy, worin der Substituent Methyl, Halogen oder Methoxy ist, Amino, Niedrigalkylamino, Di-niedrigalkylamino, Aryl-niedrigalkylamino oder Hydroxyamino sind,

$R^1$ Wasserstoff, Alkyl mit 1 bis 20 Kohlenstoffatomen, das verzweigte cyclische und ungesättigte Alkylgruppen umfaßt, substituiertes Niedrigalkyl, worin der Substituent Halogen, Hydroxy, Niedrigalkoxy, Aryloxy, Amino, Niedrigalkylamino, Di-niedrigalkylamino, Acylamino, Arylamino, Guanidino, Imidazolyl, Indolyl, Mercapto, Niedrigalkylthio, Arylthio, Carboxy, Carboxamido, Carbo-niedrigalkoxy ist, Phenyl, substituiertes Phenyl, worin der Substituent Niedrigalkyl, Niedrigalkoxy oder Halogen ist, Ar-niedrigalkyl oder Heteroar-niedrigalkyl, Ar-niedrigalkenyl oder Heteroar-niedrigalkenyl, substituiertes Ar-niedrigalkyl, substituiertes Heteroar-niedrigalkyl, substituiertes Ar-niedrigalkenyl oder substituiertes Heteroar-niedrigalkenyl, worin der Substituent Halogen oder Dihalogen, Niedrigalkyl, Hydroxy, Niedrigalkoxy, Amino, Aminomethyl, Acylamino, Di-niedrigalkylamino, Niedrigalkylamino, Carboxyl, Halogen-niedrigalkyl, Cyano oder Sulfonamido ist; Ar-niedrigalkyl oder Heteroarniedrigalkyl, das am Alkylteil durch Amino oder Acylamino substituiert ist, bedeutet,

$R^2$ und $R^7$ Wasserstoff oder Niedrigalkyl bedeuten,

$R^3$ Wasserstoff, Niedrigalkyl, Phenyl-niedrigalkyl, Aminomethylphenyl-niedrigalkyl, Hydroxyphenylniedrigalkyl, Hydroxy-niedrigalkyl, Acylamino-niedrigalkyl, Amino-niedrigalkyl, Dimethylaminoniedrigalkyl, Halogen-niedrigalkyl, Guanidino-niedrigalkyl, Imidazolyl-niedrigalkyl, Indolyl-niedrigalkyl, Mercapto-niedrigalkyl und Niedrigalkylthio-niedrigalkyl ist,

$R^4$ Wasserstoff oder Niedrigalkyl ist,

$R^5$ Wasserstoff, Niedrigalkyl, Phenyl, Phenyl-niedrigalkyl, Hydroxyphenyl-niedrigalkyl, Hydroxyniedrigalkyl, Amino-niedrigalkyl, Guanidino-niedrigalkyl, Imidazolyl-niedrigalkyl, Indolylniedrigalkyl, Mercaptoniedrigalkyl oder Niedrigalkylthio-niedrigalkyl ist,

$R^4$ und $R^5$ miteinander unter Bildung einer Alkylenbrücke mit 2 bis 4 Kohlenstoffatomen, einer Alkylenbrücke mit 2 bis 3 Kohlenstoffatomen und einem Schwefelatom, einer Alkylenbrücke mit 3 bis 4 Kohlenstoffatomen, die eine Doppelbindung oder eine Alkylenbrücke wie oben enthält, substituiert durch Hydroxy, Niedrigalkoxy, Niedrigalkyl oder Di-niedrigalkyl, verbunden sein können,

und die pharmazeutisch annehmbaren Salze davon.

Bevorzugte ACE-Hemmer im einzelnen sind Captopril (SQ-14225), Enalapril (MK-421), Lisinopril (MK-521) und Pivopril (Pivalopril, RHC-3659), weiterhin Alacepril (DU-1219), Ancovenin, Cilazapril (Ro-31-2848), Cilazaprilat, Delapril (Rev 6000A), Enalaprilat (MK-422), Foroxymithine, Fosinopril (SQ-28555), Indalapril (REV-6000A), Indolapril (CI-907), Nicotianamin, Pentopril (CGS-13945), Perindopril (S-9490-3), Phenacein, Pivopril, Quinapril (CI-906), Ramipril (Hoe-498), Rentiapril (SA-446), Spirapril, Teprotid, Trandolapril, Zofenopril (SQ-26991), 15-B-2, BRL-36378, CGS-13928-C, CGS-14824-A, CGS-16617, CI-908, CI-925, CL-242817, CV-3317, EU 5476, K-26, L-681176, MC-838, MS-41, Sch-33844, SQ-26900, Wy-44221, Y-108, YS-980 (SA-291).

Gleichzeitige Gabe von ACE-Hemmern verstärkt bemerkenswerterweise deutlich die blutdrucksenkende Wirkung der genannten Kaliumkanalaktivatoren. Dieser Effekt kann z. B. in Standardtests an narkotisierten oder wachen Ratten, Hunden, Katzen, Affen oder Minischweinen ermittelt werden, z. B. nach Methoden, wie sie in der EP-A2-02 71 271 beschrieben sind.

Die neue pharmazeutische Zubereitung kann hergestellt werden, indem man (mindestens) eine Verbindung der Formel I und/oder (mindestens) eines ihrer physiologisch unbedenklichen Salze und (mindestens) einen ACE-Hemmer zusammen mit mindestens einem festen, flüssigen oder halbflüssigen Träger- oder Hilfsstoff in eine geeignete Dosierungsform bringt. Die so erhaltenen Zubereitungen können als Arzneimittel in der Human- oder Veterinärmedizin, insbesondere bei der Senkung des Blutdrucks, eingesetzt werden. Als Trägersubstanzen kommen organische oder anorganische Stoffe in Frage, die sich für die enterale (z. B. orale oder rektale), parenterale oder topische Applikation eignen und mit den neuen Verbindungen nicht

reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate, für die topische Anwendung Salben, Cremes oder Pflaster. Die Wirkstoffe können auch lyophilisiert und die erhaltenen Lyophilisate z. B. zur Herstellung von Injektionspräparaten verwendet werden. Die Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können auch weitere Wirkstoffe enthalten, z. B. andere blutdrucksenkende oder diuretisch wirkende Stoffe.

Die erfindungsgemäßen Zubereitungen werden in der Regel zur Therapie und/oder Prophylaxe von Störungen des cardiovasculären Systems, insbesondere dekompensierter Herzinsuffizienz, Angina pectoris, peripheren oder cerebralen Gefäßerkrankungen sowie Krankheitszuständen, die mit Bluthochdruck verbunden sind, in Analogie zu bekannten blutdrucksenkend wirksamen Substanzen, insbesondere den ACE-Hemmern selbst, verabreicht. Die Dosierungen der Verbindungen der Formel I bzw. ihrer Salze liegen vorzugsweise zwischen etwa 0,01 mg und 50 mg, insbesondere 0,02 und 5 mg, ganz besonders bevorzugt zwischen 0,1 und 1 mg pro Dosierungseinheit. Die ACE-Hemmer werden vorzugsweise in Dosierungen zwischen etwa 2 und etwa 500 mg, insbesondere zwischen 5 und 200 mg pro Dosierungseinheit verwendet. Bevorzugte Dosierungen von Captopril liegen zwischen 5 und 200, insbesondere zwischen 25 und 100 mg, von Enalapril zwischen 2 und 30, insbesondere zwischen 10 und 20 mg pro Dosierungseinheit. Die tägliche Dosierung der Verbindungen der Formel I bzw. ihrer Salze liegt vorzugsweise zwischen etwa 0,0001 und 1, vorzugsweise zwischen 0,0002 und 0,1 mg/kg Körpergewicht, diejenige der ACE-Hemmer vorzugsweise zwischen etwa 0,04 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Bestandteile der neuen pharmazeutischen Zubereitung werden vorzugsweise kombiniert verabreicht. Sie können aber auch einzeln gleichzeitig oder aufeinander-folgend verabreicht werden.

Beispiel 1: Tabletten

Ein Gemisch von 20 g Ia, 0,5 kg Captopril, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 1 mg Ia und 25 mg Captopril enthält.

Beispiel 2: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt ein gesiebtes Gemisch von 30 g Ib, 1 kg Enalaprilhydrogenmaleat und 6 kg Lactose in üblicher Weise in Hartgelatinekapseln, so daß jede Kapsel 0,3 mg Ib und 10 mg Enalaprilhydrogenmaleat enthält.

Beispiel D: Ampullen

Eine Lösung von 2 g Ic und 0,1 kg Cilazapril-Natriumsalz in 30 l zweifach destilliertem Wasser wird steril filtriert, in Ampullen abgefüllt, unter sterilen Bedingungen lyophilisiert und steril verschlossen. Jede Ampulle enthält 0,1 mg Ic und 5 mg Cilazapril-Natriumsalz.

## Ansprüche

1. Pharmazeutische Zubereitung enthaltend einen Kaliumkanalaktivator und einen ACE-Hemmer, dadurch gekennzeichnet, daß sie einen Kaliumkanalaktivator der Formel I enthält:

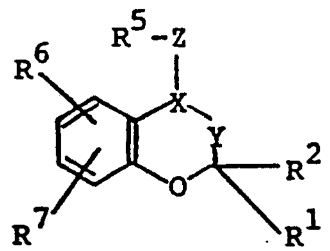

I

worin

X-Y =CH-$CHR^8$-, =$CR^4$-$CR^3$A- oder =CH-CA(OA)-, sowie, falls der Rest $R^5$ weder vollständig noch partiell hydriert ist, auch =$CR^4$-$CHR^3$- oder =CH-CH(OA)-,

$R^1$ A,

$R^2$ und $R^8$ jeweils H oder A,

$R^1$ und $R^2$ zusammen auch Alkylen mit 3-6 C-Atomen,

$R^3$ OH oder OAc,

$R^4$ H,

$R^3$ und $R^4$ zusammen auch eine Bindung,

$R^5$ einen unsubstituierten oder einen ein-oder zweifach durch A, F, Cl, Br, J, OH, OA, OAc, $NO_2$, $NH_2$, AcNH, HOOC und/oder AOOC substituierten Pyridyl-, Pyridazinyl-, Pyrimidinyl-, Pyrazinyl-, Oxo-dihydropyridyl-, Oxo-dihydro-pyridazinyl-, Oxo-dihydro-pyrimidinyl-, Oxo-dihydro-pyrazinyl-, 3H- oder 5H-2-Pyrrolinon-1-yl-, 2H-1-Isochinolinon-2-yl-, 2H-1-Phthalazinon-2-yl-, 3H-4-Chinazolinon-3-yl- oder 1H-2-Thiopyridon-1-yl-rest, wobei diese Reste auch vollständig oder partiell hydriert sein können,

$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyanalkyl, A-C(=NOH) oder A-C(=$NNH_2$),

Z eine Bindung, O, S oder NH,

$R^7$ auch Pyridyl, Pyridazinyl, Pyrimidinyl oder Pyrazinyl,

A Alkyl mit 1-6 C-Atomen (wobei mehrere Gruppen A unabhängig voneinander verschiedene Alkylgruppen bedeuten können),

-alkyl- Alkylen mit 1-6 C-Atomen und

Ac Alkanoyl mit 1-8 C-Atomen oder Aroyl mit 7-11 C-Atomen

bedeuten,

und/oder eines seiner physiologisch unbedenklichen Salze.

2. Verfahren zur Herstellung einer pharmazeutischen Zubereitung nach Anspruch 1, dadurch gekennzeichnet, daß man eine Verbindung der Formel I und/oder eines ihrer physiologisch unbedenklichen Salze und einen ACE-Hemmer zusammen mit mindestens einem festen, flüssigen oder halbflüssigen Träger- oder Hilfsstoff in eine geeignete Dosierungsform bringt.

3. Verwendung einer pharmazeutischen Zubereitung nach Anspruch 1 bei der Senkung des Blutdrucks.